# EUROPEAN PATENT APPLICATION

(11) **EP 0 890 639 A2**
(43) Date of publication of application: **13.01.1999**
(21) Application number: 98112742.6
(22) Date of filing: 09.07.1998
(51) Int. Cl.: C12N 15/12, C07K 14/47

(54) **BMP2-induced cDNA and its use**

(30) Priority: 09.07.1997 EP 97111602
(71) Applicant: Gesellschaft für biotechnologische Forschung mbH (GBF), 38124 Braunschweig (DE)
(72) Inventor: Ahrens, Marion, 38124 Braunschweig (DE); Bächner, Dietmar, 38124 Braunschweig (DE); Hoffmann, Andrea, 38124 Braunschweig (DE); Lauber, Jörg, 38124 Braunschweig (DE); Steinert, Peter, 38124 Braunschweig (DE); Flohé, Leopold, 38124 Braunschweig (DE); Gross, Claus-Gerhard, 38124 Braunschweig (DE)
(74) Representative: Boeters, Hans Dietrich, Dr.

(57) **Abstract**

The recombinant expression of human BMP2, BMP4 - BMP7 in murine mesenchymal C3H10T½ progenitors mediates differentiation into three mesenchymal Iineages in different efficiencies: the osteogenic, the chondrogenic and the adipogenic lineage. This developmental *in vitro* model was used to identify and to characterize cDNAs involved during the manifestation of these lineages *in vivo*. By subtractive cloning an as yet undescribed cDNA, **29A** has been cloned which encodes a putative secreted factor which is expressed in developing osteo- /chondrogenic tissues of vertebrae, ribs, tooth and the limb bud.

## Description

### Introduction

During embryonic development, the vertebrate skeletal elements arise from mesenchymal cell-condensations forming cartilage or bone, eventually. Recent studies suggest that bone morphogenetic proteins (BMPs) may play a crucial role during the formation of skeletal condensations and the onset of differentiation from mesenchymal progenitors. The mammalian bone morphogenetic proteins (BMPs) were originally purified and characterized from adult bone on the basis of their ability to induce a cascade of events leading to ectopic bone formation if implanted subcutaneously or at intra-muscular sites.

Molecular cloning of these genes and biochemical characterizations established BMPs (with the exception of BMP1) as a family of proteins which are generated from dimeric precursors proteolytically processed to 25,000-30,000 Mᵣ homo- or heterodimers belonging to the transforming growth factor β (TGF-β) superfamily. Members of this family can be classified to the degree of amino acid identity of their C-terminal domains. The BMPs also share a high identity to other closely related proteins which have been characterized in *Xenopus* and *Drosophila* like the *decapentaplegic* (*dpp*) gene, the latter being involved both in dorsoventral body patterning and in imaginal disk formation (Irish and Gelbart. 1987; Ferguson and Anderson, 1992). The BMPs are also related to Vgl. which in *Xenopus* has been postulated toplay a role in embryonic development and mesoderm specification (Blessing et al., 1993; Weeks and Melton, 1987; Lyons et al., 1989b: Lyons et al., 1989a). While the homogenous inactivation of the murine BMP4 as well as the BMP type Ia receptor genes results in an early einbryonic lethality consistent with a putative role in early mesoderm formation (Winner et al., 1995; Mishina et al., 1995), BMP7 null mice exhibit only relatively mild skeletal abnormalities affecting mesenchymal condensations rather than chondrogenic differentiation (Karsenty et al., 1996; Hofmann et al., 1996).

BMPs induce condensations and chondrogenesis in primay cells and cell lines derived from limb buds. The potency of the various BMPs (BMP2-7) differ in these primary systems but in all cases they directly mediate chondrocytic differentiation.

The murine fibroblastic C3H10T½ cell line which has been established from an early-stage mouse embryo represents a relatively early stage of mesenchymal cell determination with the ability to differentiate into myoblasts, adipocytes, chondrocytes and osteoblasts (Reznikoff et al., 1973; Taylor and Jones. 1979; Wang et al., 1993; Ahrens et al., 1993) Their responsiveness towards TGF-β and BMP-treatment make this line a useful model system to explore the involvement of factors in various mesenchymal differentiation processes. BMP2 and BMP4 possess the ability in mesenchymal progenitor C3H10T½ cells to mediate the differentiation into chondrocytes, osteoblasts and adipocytes (Wang et al., 1993; Ahrens et al., 1993). The extension of the analysis onto other members of the family BMP5-7 in this study shows that all BMPs investigated possess the potency to mediate differentiation into three mesenchymal cell types. albeit in largely varying efficiencies. To assess the value of the C3H10T½ *in vitro* model as a model for BMP-mediated mesenchymal differentiation we performed subtractive cloning analysis for BMP-upregulated genes in this system and characterized their expression in the embryonic development in the mouse by *in situ* hybridization We were able to isolate BMP-upregulated genes typical for the osteo- /chondrogenic and adipogenic development. In addition we characterized BMP-upregulated genes and cDNAs which are unknown or as yet have not been assigned to BMP-mediated mesenchymal development. Among these genes is a new putative secreted factor **29A** which is expressed in osteoblasts, in murine embryonic development in in precartilage condensation and during limb and tooth development.

### Materials and Methods

### Cell Lines, culture conditions and transfection experiments

The features of BMP2 and BMP4 transfected C3H10T½ cells have been described by Ahrens et al. ( 1993). Human BMP2 and BMP4 are constitutively transcribed by the LTR of the myeloproliferative sarcoma virus (MPSV). Human versions of BMP5, BMP6 and BMP7 have been described in Wozney et al. (1989) and incorporated in the expression vector described before. Transfection was performed by calcium-phosphate precipitation. Control or BMP-transfected C3H10T½ cells were selected by cotransfection with pSV2pac mediating resistance against puromycin (5µg/ml). Puromycin-resistent colonies were subcultivated and selection-pressure was maintained during the entire cultivation period to follow. If not stated otherwise cells were plated at a density of 5.000 cells /cm². Cells were routinely grown in Dulbecco's modified Eagle's medium (DMEM) supplemented with 10% fetal calf serum. After reaching confluence (arbitrarily termed day 0) 50 µg/ml ascorbic acid and 10 *mM* β-glycerophosphate were added as specified in the protocol of Owen et al., (1990) for the cultivation of native osteoblast-like cells. Primary osteoblast-like cells were isolated from the calvariae of 5 day old mice (NMRI) by sequential collagenase digestion and cultivated as described (Owen et al., 1990).

### mRNA analysis

C3H10T½ cells harboring the expression vectors were cultivated as described above. Cells were harvested at the indicated time intervals and total RNA was isolated by guanidinium/CsCl step-gradients. Total cellular RNA (10µg) was separated electrophoretically in a 2.2 M formaldehyde -1.2% agarose gel and transferred to nitrocellulose. Hybridization was carried out with nick-translated ³²P-labelled gene-specific DNA probes.

### Histological methods and verification of cellular phenorypes

Osteoblasts exhibit a stellate morphology displaying high levels of alkaline phosphatase activity which was visualized by cellular staining with α-naphthyl-phosphate and Fast Red. Deposition of mineral was monitored by von Kossa-staining method (Owen et al., 1990). Osteogenesis was also investigated by northern analyses with hyhridization probes typical or specific for the osteogenic developmental sequence namely collagen I, osteopontin, osteonectin and osteocalcin. In addition, the up-regulation of the parathyroid hormone receptor (PTH/PTHrP-receptor) at the onset of the osteogenic developmental sequence was studied by northern analyses. Adipocytes were identified morphologically as oil-droplet filled cells and/or by staining with Oil Red O. Marker genes for adipocytes were the lipoprotein lipase and ap2. Chondrocytes were identified by staining with Alcian Blue at pH 2.5. They also displayed alkaline phosphatase activity but in comparison with osteoblasts differed in intensity and a rounded cell morphology. Marker genes for chondrocyes was collagen II.

### 5'- and 3'- extension of cDNA clones by the RACE - technology (Rapid Amplification of cDNA nds)

To complete the **29A** cDNA 5'-RACE was performed essentially as described (Frohmann. 1990). Random primers were removed from the first strand cDNA preparation by gel purification (Jetsorb-Kit. Genomed). and a dA-tail was added to the 3'-end of the single-stranded DNA by terminal transferase in a 10 µl-reaction containing 200 mM potassium cacodylate, 25 mM Tris pH 6.6, 0.25 mg/ml bovine serum albumin. 1.5 mM CoCl₂, 0.5 mM dATP and 10 U enzyme (25 min. 37°C). After phenolization and precipitation, the first PCR-amplification was performed using the primers R₀-R₁-(dT₁₇) and 3'-reverse primer (30 pmol each) under the same temperature conditions as detailed for RT-FCR except that 50°C was used for annealing. The region corresponding to the desired band was eluted from a low-melting agarose gel and reamplified using the primers R₀ and a new nested 3'- reverse primer (N₁; 30 pmol each). For the 3'-RACE, total RNA was reverse transcribed using 2.5 µg Oligo-dT₁₂₋₁₈ (Gibco-BRL) as described above. Double-stranded cDNA was then immediately synthesized by adding 30 pmol 5'-primer and fresh dNTPs to the reaction mixture (1h, 37°C). The sample was gel-purified and the first PCR-amplification was performed using the primers R₀-R₁-(dT₁₇) and a new nested 5'-primer (N₂; 30 pmol each) followed by a second PCR with the primers R₀ and

### Subtractive cloning

Subtractive cDNA cloning was performed after a modified protocol . *mRNA preparation*. BMP-2 and untransfected C3H10T½ cells were cultivated as described above. Eight days after reaching confiuence cells were harvested and total RNA was isolated by guanidinium/CsCl step gradients. mRNA was purified from total RNA by oligo(dT)-cellulose chromatography. *cDNA synthesis and library construction*. mRNA (2.5 µg) from BMP-2-transfected (target) and control C3H10T½ cells (driver) were reverse transcribed with Moloney murine leukemia virus reverse transcriptase (BRL) in the presence of 1 µg of random hexanucleotide (Pharmacia). Second strand synthesis was performed with a commercially available cDNA Synthesis Kit (BRL). cDNA was degraded to ∼500 bp by a short ultrasonic pulse (3 x 5 sec) in a Branson sonifier (250 watts). The latter step is thought to prevent a PCR-dependent amplification of short cDNA sequences in the subtractive cloning procedure. After sonification staggered ends were filled up by treatment with T4-DNA polymerase. The driver and the target cDNAs were ligated to different oligonucleotides harboring a *Eco* RI restriction or a *Hind* III restriction site. respectively (Duguid and Dinauer (1989)). For the driver cDNAs the sequence of the oligonucleotides was :
3' ATCAGGCTTAAGTTCGTTCTC 5'
5' TAGTCCGAATTCAAGCAAGAGCACA 3'.
For the target library the sequence of oligonucleotides was:
3' TAGCAGTTCGAAGTTCAATCG 5'
5' ATCGTCAAGCTTCAAGTTAGCATCG 3'.
cDNAs were amplified by PCR (30 cycles) using the different oligonucleotides as primers. The PCR-amplification of the control cDNA was performed with 0.25 *mM* Biotin-4-dUTP as dTTP analog. The PCR-products were purified with *Strataclean* Resin from *Stratugene* and the incorporation of the biotinylated nucleotides was verified by the BluGENE nonradioactive nucleic acid detection system from BRL. The amplified cDNA libraries from both control and BMP-2-transfected C3H10T½ cells represent the starting material for the subtractive cloning procedure. *Library subtraction*. 15 µg of the biotinylated control library cDNA was mixed with 1.5 µg of the BMP-2-transfected library cDNA. In addition, 150 ng of biotinylated BMP2 cDNA was added to remove BMP-2-transcripts from the subtracted library. The mixture was denaturated and hybridized for 20 h at 68 °C. After hybridization biotinylated molecules were removed from the mixture with Dynabeads M-280 Streptavidin (DYNAL). The resulting subtracted cDNA was ethanol precipitated and hybridized with another 15 µg of biotinylated control cDNA and the cycle was repeated. The resulting cDNA was amplified by PCR (15 cycles) using the target-library specific 21-mer oligonucleotide as primer. In total six liybridization/subtraction cycles were performed. The resulting cDNAs were cleaved with *Hind* III, ligated into the eukaryotic expression vector pMBC-2T-fl and transformed into competent *E. coli* SURE cells. The colonies were transferred to nitrocellulose and a plus-minus-screening was performed using the subtrative library and the control/driver library as probes. ∼30% of the clones proved to be differentially expressed and were further characterized by DNA sequencing and northern analyses.

### Mice and RNA-in situ-hybridization

Embryos were isolated from pregnant NMRI mice at the developmental stages indicated in the text. The day of plug detection was considered to be day 0.5 post conceptionem (dpc). The embryos were fixed overnight with 4% paraformaldehyde in PBS at 4°C. For radioactive RNA-ill sitll-hybridization fixed mouse embryos and tissues were prepared for cryostat sectioning as described previously (Bächner et al., 1993). 10 µm cryosections were mounted on 3-aminopropyltriethoxysilane coated slides. Antisense and sense riboprobes were generated by RNA in vitro transcription with [³⁵S]-dUTP to a specific activity of >10⁹ dpm/µg. Probe length was reduced to 150 - 200 nucleotides by alkaline hydrolysis (Cox et al., 1984). The slides were prehybridized at 54°C in a solution containing 50% formamide, 10% dextrane sulfate, 0.3 M NaCl, 10 mM Tris. 10 mM sodium phosphate pH 6.8. 20 mM dithiothreitol, 0.2x Denhardt's reagent. 0.1% Triton X-100, 1.25 mg/ml yeast RNA. and "cold" 0.1 mM [S]-dUTP. For hybridization, 80000 dpm/µl [³⁵S]-dUTP labelled probe RNA was added to the hybridization mix, and the hybridization was continued at 54°C for 16 h in a humid chamber. The slides were washed in hybridization salt solution to which ditniothreitol was added. After RNase A digestion (40 µg/ml) the slides were washed for 30 min at 37°C with 2x SSC, 0.1% (w/v) SDS and 30 min with 0.1x SSC and dehydrated by increasing concentrations of ethanol The slides were coated with Ilford K5 photoemulsion for autoradiography. After 1 to 4 weeks of exposure at 4°C. depending on cDNA analysed, the slides were developed and stained with Giemsa solution. The embryos and sections were analyzed with bright- and dark-field illumination with a Zeiss SV11 stereo-microscope and an Zeiss Axiophot microscope and photographed using Kodak Ektachrome 320T or Agfa Ortho 25 film. Figures were prepared using a Polaroid SlideScanner together with Adobe Photoshop and Adobe PageMaker software with a Windows NT-computer device.

### Results

### Expression of BMPs in C3H10T½ mesenchymal precursor celis

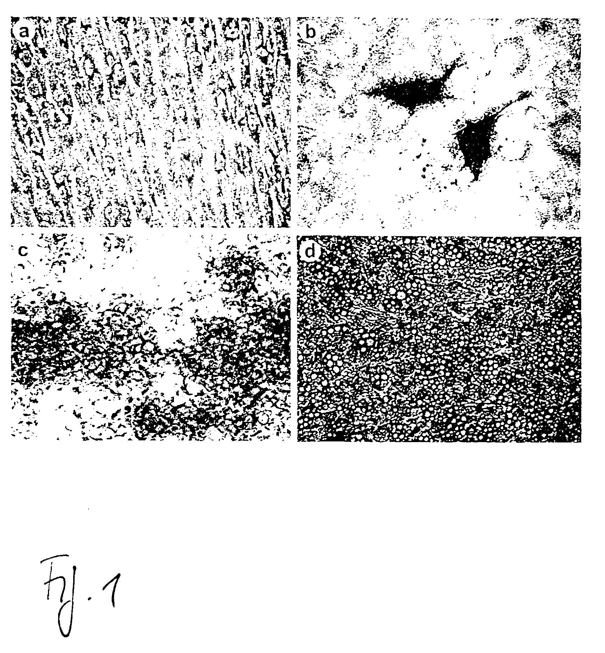

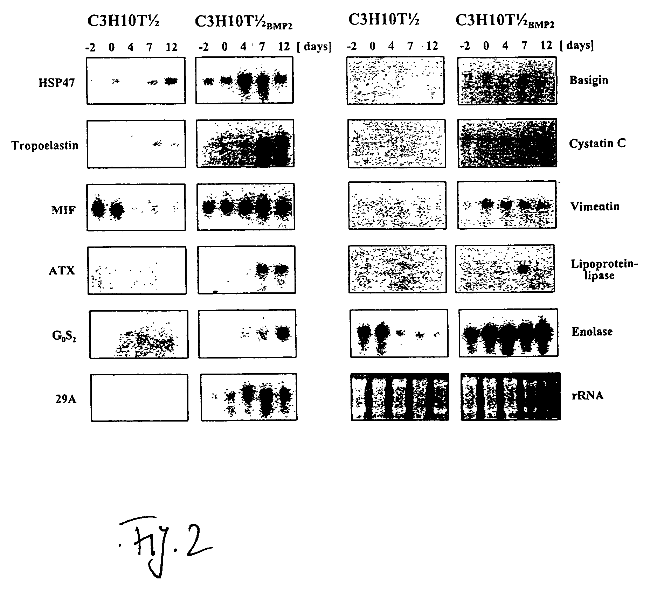

To compare the *in vitro* characteristics of BMP-mediated differentiation with other members of the BMP-family the cDNAs encoding the human version for BMP2-7 (without BMP3) were integrated into an expressionvector (Ahrens et al., 1993) and transfected into C3H10T½ rnesenchymal precursor cells. 5.000-10.000 transfectants per individual member of the BMP-family were pooled and investigated further. After reaching confluence all BMP-transfected cells started to grow in multilayer and extensive matrix production accompanies a development into three distinct mesenchymal lineages: the adipogenic, the osteogenic and the chondrogenic (Fig. 1) (Ahrens et al., 1993: Wang et al., 1993).

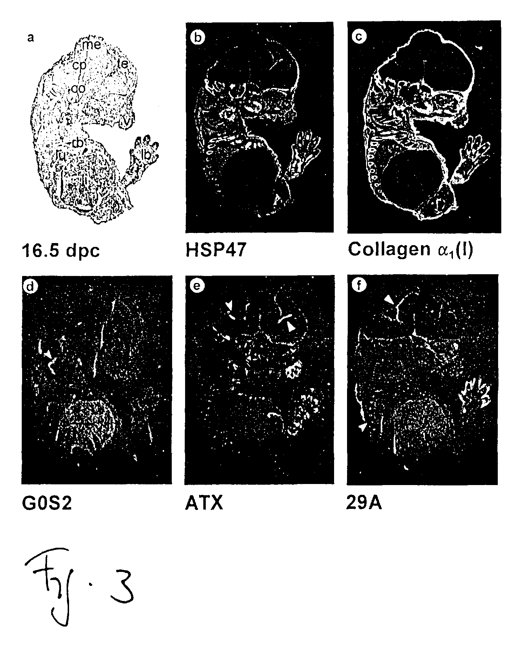

Although in general the differentiation in osteoblasts. chondrocytes and adipocytes was observed for all BMPs investigated on the basis of moiphological and histological criteria (Fig. 1), the ability of the various BMP-expressing C3H10T½ cells to undergo mesenchymal development within a two week cultivation period was different among the various members of the BMP-family (Table I). In all cases, the level of BMP-specific mRNA was comparable for the various BMP-expressing C3H10T½ cells. The mRNA encoding the BMPs are, in general. expressed at the highest level in actively proliferating cells up to few days after reaching confluence (Fig. 2). Secreted BMPs are difficult to monitor in the supematnat of C3H10T½ cells. We could confirm in another study by western analyses that BMPs such as BMP2 and BMP4 can only marginally be monitored in the supernatant, indicating that they are efficiently bound by extracellular matrix (Wang et al., 1993).

### Isolation of BMP-2 induced cDNAs from the mesenchymal progenitor cell line C3H10T½

We employed the subtractive cloning procedure outlined in Materials and Methods to C3H10T½ cells expressing recombinant BMP2 at 8 day post-confluence. At this stage the first moiphologiocal distinct phentoypes of the various mesenchymal lineages are monitored and, therefore, we should be able to isolate cDNAs which are involved in the determination of these lineages as well as cDNAs which could play a role in the morphological manifestation of the mesenchymal cell types. The subtractive cloning strategy involves two different PCR-primer sets for the PCR-amplification of the driver and the target library and the addition of biotinylated BMP2 cDNA to the target library to prevent the selection of cDNAs originating from recombinant BMP2-transcripts. After the subtraction procedure. *E. coli* colonies harboring the cloned cDNA fragments were transferred to nitrocellulose. and a plus-minus-screening was performed using the amplified cDNAs from the subtrative library and the control/driver library as probes. Roughly 10.000 colonies were screened and about 30 % of the clones proved to be differentially expressed. Of these 200 were further characterized by northern analyses and DNA sequencing.

Partially or complete sequencing of 21 BMP-regulated cDNAs revealed that 20 were related to known sequences and one was not (29 A) (Table II). About 50 % of the cDNAs consisted of four different sequence types (Table II). The others were represented between 1 - 4% in the subtracted library. Among the known sequences were collagens, several enzymes of the glycolytic pathway, cystatin C, vimentin, basigin, tropoelastin, migration inhibitory factor (MIF), osteopontin, lipoproteinlipase the heatshockprotein HSP-47, autotaxin (ATX) as well as one member of the family of the CCAATbinding family of transcription factors, C/EBPα. Of the former some were not previously recognized as BMP-upregulated genes, namely C/EBPα, HSP-47. MIF, csytatin C, basigin, vimentin, G0S2, tropoelastin autotaxin (ATX) and **29A**. The expression profile of these genes was characterized by northern analyses in parental and recombinant BMP2 expressing C3H10T½ cells (Fig. 2) which shows that the genes isolated by subtractive cloning were expressed predominantly at middle to late late cultivation stages (e. g. autotaxin, G0S2, tropoelastin) but some BMP-regulated mRNAs are regulated already early on in recombinant BMP-expressing cells like e. g. HSP47, vimentin and especially the undescribed cDNA **29A** which encodes a putative secreted factor (see below)(Fig. 2).

### BMP-regulated gene 29A isolated from C3H10T½ cells is involed in the development of the osteo- /chondrogenic lineage.

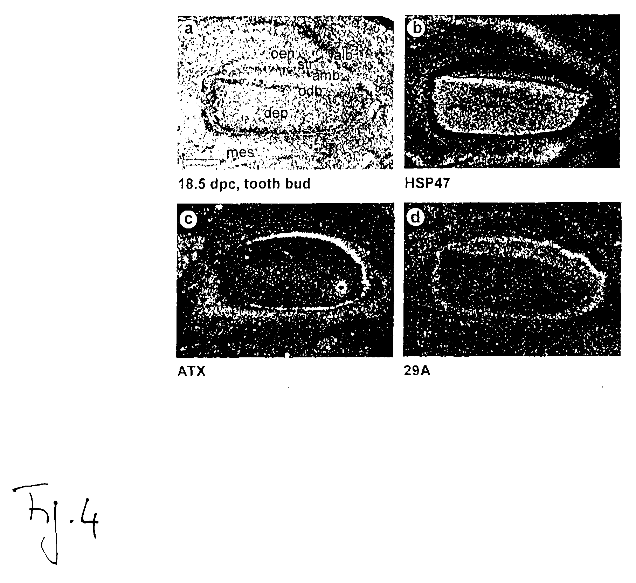

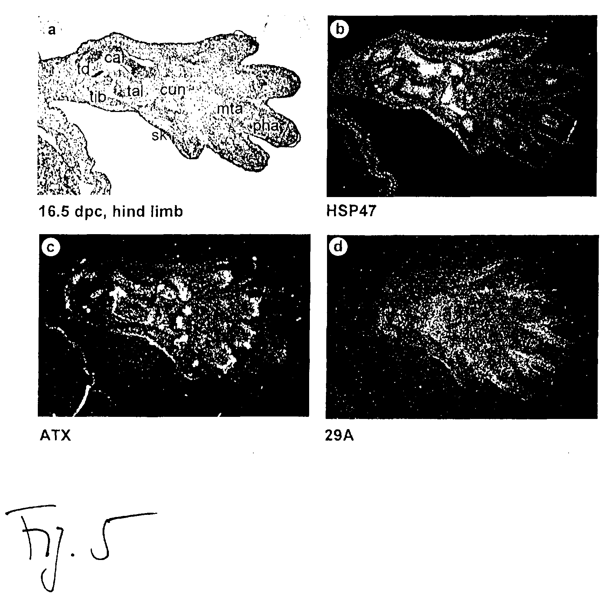

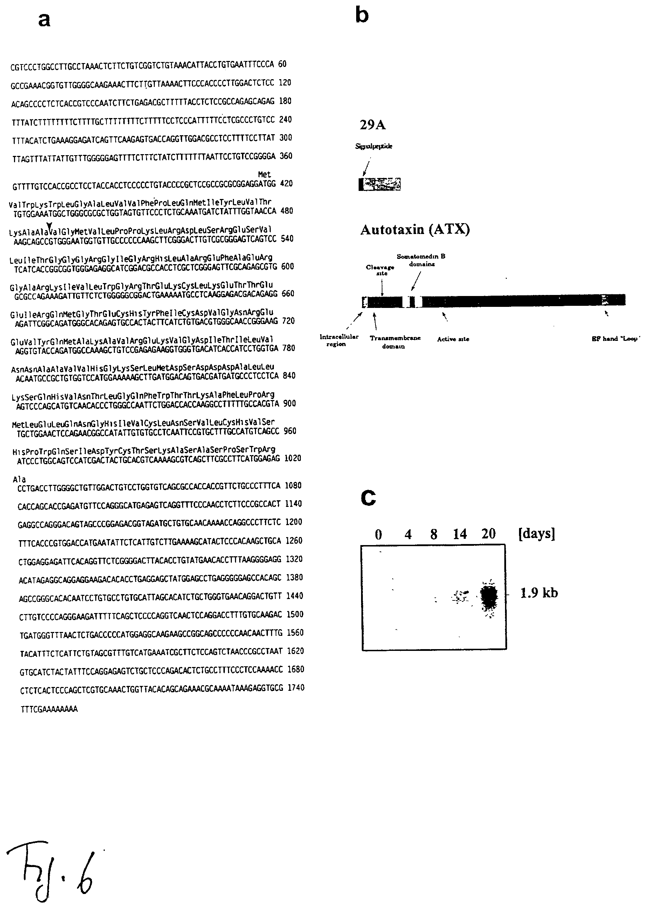

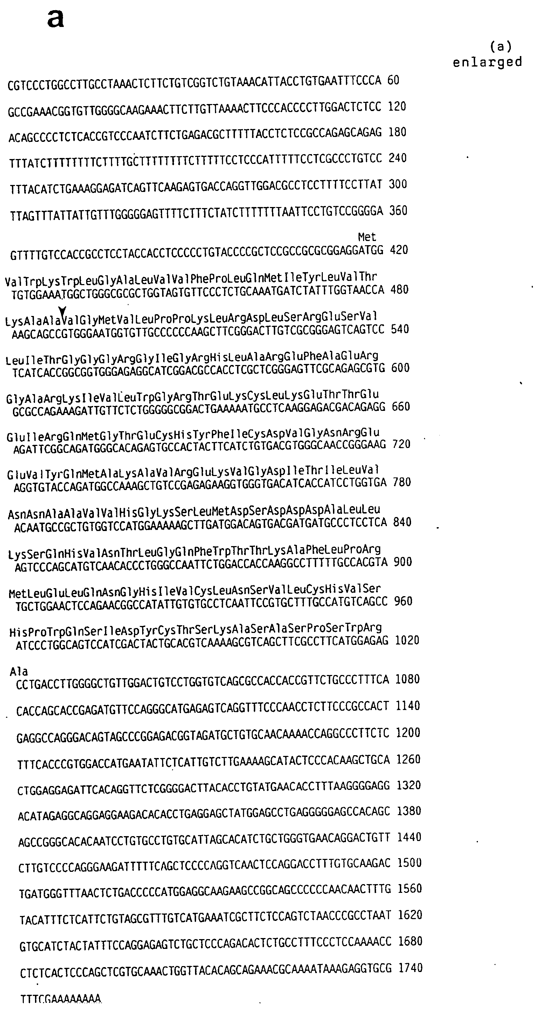

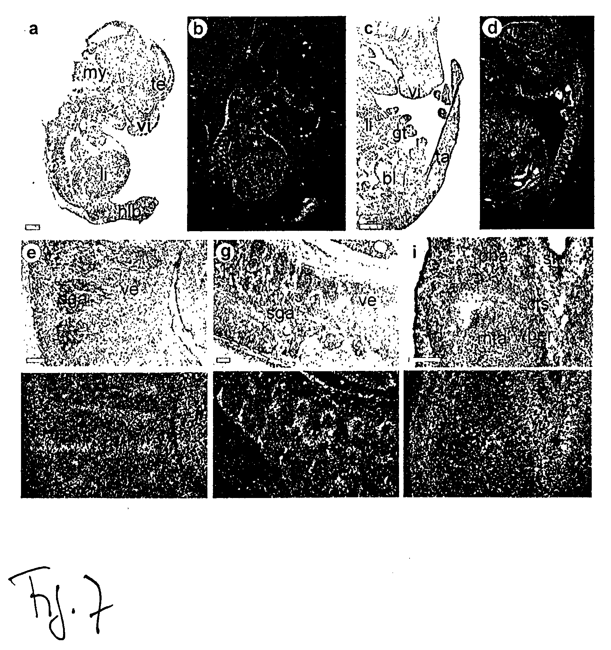

**29A** has been isolated as a 250 bp cDNA fragment hybridizing to a BMP-upregulated 1.8 kb mRNA in C3H10T½ cells (Fig. 2). The cDNA sequence has been completed by the RACE technology and confirmed by cDNA cloning. It contains several open reading frames. Already the the first translational start site obeys the Kozak rules and is followed by an open reading frame coding for a protein with a M_{w} 22 kDa (Fig 6a). **29A** seems as a secreted factor indicated by the N-terminal hydrophobic stretch of 30 aminoacid residues. The putative signal-sequence cleavage-site has been located between aminoacid positions 23-24 (Signal-program at ExPASy-Tools: ISREC, Lausanne). The protein sequence does not exhibit a significant homology to known sequences. During murine development **29A** expression is detected in a variety of mesodermal tissues. Enhanced expression is first detected in presumptive bone-forming centers of vertebrae at 12.5 dpc (Fig. 7e. t) and later gets restricted to the perichondreum of the forming vertebrae (Fig. 7c.d.g.h). In midgestation development expression is further detected in the ribs. toothbud and forming vibrisae (Fig. 3f; 5d. 7c.d). During limb development enhanced expression gets restricted to the perichondreum and cormective tissue sheet of the forming metatarsales and phalanges (Fig. 4d). In late tooth development **29A** expression is restricted to the outer enamel epithelium and the ameloblasts (Fig. 5d). **29A** is also expressed in primary murine osteoblasts from 5-day old mice suggesting a potential regulatory role for this protein in bone growth (Fig. 6c).

### Disscussion

### BMP2 upregulated cDNAs in C3H10T½ cells

A new finding was the unknown BMP2-upregulated gene **29A** which is expressed early in C3H10T½ cells and primary osteoblasts. In the latter cells its expression increases at late stages of of the development *in vitro* which is comparable with many osteogenic marker genes(Fig. 6c). The putative secreted factor **29A** could be one of the many growth factors which influence the initiation and the manifestation of the bone and/or cartilage phenotype. Such factors exist in a wide variety involving very different factors like e. g. insulin-like growth factors (IGFs), parathyroid hormone related protein (PTHrP) and indian hedgehg (IHH). At present the role of **29A** is determined in several differentiation systems. Such studies also show that a homologus factor is found in the human system.

### Refernces

Ahrens, M.. T. Ankenbauer. D. Schroder. A. Hollnagel, H. Mayer. and G. Gross. 1993. Expression of Human Bone Morphogenetic Proteins - 2 or - 4 in Murine Mesenchymal Progenitor C3H10T½ Cells Induces Differentiation into Distinct Mesenchymal Cell Lineages. *DNA Cell Biol.* 12:871-880.

Bächner. D.. A. Manca, P. Steinbach, D. Wöhrle, W. Just. W. Vogel, H. Hameister, and A. Poustka. 1993. Enhanced expression of the murine FMR1 gene during germ cell proliferation suggests a special function in both the male and female gonad. *Hum. Mol. Genet*. 2:2043-2050.

Blessing, M.. L. B. Nanney, L. E. King, C. M. Jones. and B. L. M. Hogan. 1993. Transgenic mice as a model to study the role of TGF-β-related molecules in hair follicles. *Genes Dev.* 7:204-215.

Cox. K. H.. D. V. DeLeon, L. M. Angerer, and R. C. Angerer. 1984. Detection of mRNAs in sea urchin embryos by in situ hybridization using asymmetric RNA probes. *Dev. Biol*. 101:485-502.

Ferguson. E. L. and K. V. Anderson. 1992. Localized enhancement and repression of the activity of the TGF-β family member, *decapentaplegic*, is necessary for dorsal-ventral pattern formation in the *Drosophila* embryo. *Development* 114:583-597.

Frohmann. M. A. 1990. Rapid amplification of cDNA ends (RACE): user-friendly cDNA cloning. *Amplifcations* 5:11-15.

Hofmann, C., G. B. Luo, R. Balling. and G. Karsenty. 1996. Analysis of limb patterning in BMP-7-deficient mice. *Dev. Genet.* 19:43-50.

Irish, V. F. and W. M. Gelbart. 1987. The decapentaplegic gene is required for dorsal-ventral patterning of the *Drosophila embryo*. Genes *Dev*. 1:868-879.

Karsenty, G., G. B. Luo, C. Hofmann, and A. Bradley. 1996. BMP 7 is required for nephrogenesis, eye development, and skeletal patterning. *Ann*. *NY Acad. Sci*. 785:98-107.

Lyons, K. M., J. L. Graycar, A. Lee, S. Hashmi, P. B. Lindquist. E. Y. Chen, B. L. M. Hogen, and R. Derynck. 1989a. Vgr-1, a mammalian gene related to xenopus Bg-1 is a member of the transforming growth factor β gene superfamily. *Proc. Natl. Acad. Sci. USA* 86:4554-4558.

Lyons, K. M., R. W. Pelton, and B. L. M. Hogan. 1989b. Patterns of expression of murine Vgr- 1 and BMP-2a RNA suggest that transforming growth factor-β-like genes coordinately regulate aspects of embryonic development. *Genes Dev*. 3:1657-1668.

Mishina, Y., A. Suzuki, N. Ueno. and R. R. Behringer. 1995. Bmpr encodes a type I bone morphogenetic protein receptor that is essential for gastrulation during mouse embryogenesis. *Genes Dev*. 9:3027-3037.

Owen, T. A., M. Aronow, V. Shalhoub, L. M. Barone, L. Wilming, M. S. Tassinari, M. B. Kennedy, S. Pockwinse, J. B. Lian, and G. S. Stein. 1990. Progressive Development of the rat osteoblast phenotype in vitro, reciprocal relationships in the expression of genes associated with osteoblast proliferation and differentiation during formation of the bone extracellular matrix. *J. Cell. Physiol.* 143:420-430.

Reznikoff. C. A.. D. W. Brankow. and C. Heidelberger. 1973. Establishment and characterization of a cloned cell line of C3H mouse embryo cells sensitive to postconfluence inhibition of division. *Cancer Res*. 33:3231-3238.

Taylor. S. M. and P. A. Jones. 1979. Multiple new phenotypes induced in 10T½ and 3T3 cells treated with 5-azacytidine. *Cell* 17:771-779.

Wang, E. A., D. I. Israel, S. Kelly, and D. P. Luxenberg. 1993. Bone-Morphogenetic protein causes commitment and differentiation in C3H10T½ and 3T3 cells. *Growth Factors* 9:57-71.

Weeks. D. L. and D. A. Melton. 1987. A maternal mRNA localized to the vegital hemisphere in xenopus eggs codes for a growth factor related to TGF β. *Cell* 51:861-867.

Winner. G., M. Blessing, P. A. Labosky. and B. L. M. Hogan. 1995. Bone-morphogenetic protein-4 is required for mesoderm formation and patterning in the mouse. *Genes Dev.* 9:2105-2116.

Wozney, J. M. 1989 Bone morphogenetic proteins. *Prog. Growth Factor Res.* 1:267-280.

### Legend to Tables

**Table I.** Differentiation potential of C3H10T½ cells stably expressing members of the BMP-family. Numbers represent cells/cm². Cells were grown in 4.5 cm² wells. The total number of cells was determined in acoulter counter after treatment of the dense adherent cellular layer with collagenase. The values represent mean values of three independent cultivations. The number of osteoblast-like cells werewas evaluated by assessing the number of alkaline phosphatase positive colonies. The number of chondroblastic cells was determined after staining with Alcian Blue. Adipocytes were identified moiphologically or stained with Oil Red O. The multilayer growth obstructed exact evaluation ofthe mesenchymal lineages at later stages of cultivation. Therefore. the number of osteoblast-like and chondroblastic cells represent approximated values of three independent cultivations. The number of adipocytes which heavily covered the cells was not determined.

**Table II.** BMP-regulated genes isolated by subtractive cloning from recombinant BMP2-expressing C3H10T½ cells. Subtractive cloning is detailed in Materials and Methods. ∼200 BMP-upregulated cDNAs afte subtrative cloning were investigated in northern analyses and by sequencing. The percentages indicate the representation of the respective cDNAs in the subtractive cDNA library. The other cDNAs are represented between ∼1%-4% in the library. * indicate members of the C/EBP-family which have not been detected by subtractive libray but were found to be upregulated by BMP during the course of this study (see also Fig.4)

### Legends to Figures:

**Fig. 1.** Histological analysis of mesenchymal development in C3H10T½ cells. a. C3H10T½ cells stably transfected with the expressionvector pMT7T3 (10 days after reaching confluence) b. Alkaline phosphatase positive osteoblast-like cells in C3H10T½ cells stably expressing BMP2 (10 days post-confluence) c. Alcian blue positive. chondrocyte-like cells in C3H10T½ cells stably expressing BMP7 (12 days post-confluence).d. oil-droplet filled adipocyte-like cells in C3H10T½ cells stably expressing BMP4 (12 days post-confluence).
**Fig. 2** Northern analyses of BMP2-regulated genes isolated by subtractive cloning from C3H10T½ cells. Northern analyses were performed as describesd in Fig. 2 and Materials and Methods.
**Fig. 3.** Expression of BMP-regulated genes in murine midgestation development. Bnght (a) and dark field (b-f) images of consecutive parasagittal cryosections of a 16.5 dpc mouse embryo hybridized with a HSP47 (b), collagen α₁ (I), G0S2 (d), ATX (e), and **29A** (f) antisense riboprobe are shown. HSP47 and collagen (I) are coexpressed in many cartilage tissues, for example the ribs and cranial bones (b,c). Expression of G0S2 is in contrast restricted to forming brown adipose tissue (arrow in d), a lower level of expression is also visible in the liver (d). ATX expression is most prominent in the mesenchyme surrounding the forming vibrissae, the choroid plexus epithelia of the IVth. (arrow in e) and Ist (arrowhead in e) ventricle of the brain. Enhanced expression of ATX is in addition vissible in different cartilage tissues, for examples within the hind limb, ribs, and cranial bones (e). Expression of **29A** is most prominent in the hind limb, ribs (arrow in f), and epithelia of the brain (arrowhead in f). cp. choroid plexus; co, cochlea: li, liver; lu, lung; me, mesencephalon; rb, rib; te, telencephalon: vi. vibrissae. Bar, 1 mm.
**Fig. 4.** Expression of BMP-regulated genes within 16.5 dpc hind limb, Higher magnifications of the hind limb of Fig. 5 are shown. Wheras HSP47 expression within the developing limb is most prominent in the cartilage of the forming bones (b), expression of ATX is restricted to the joint regions of the forming bones (c). and expression of **29A** most prominent in the perichondreum and adjacent connective tissue sheet of the metatarsales and phalanges (d). cal, calcaneum; cun, cuneiforme, mta, metatarsale; pha, phalange; sk, skin; tal talus; tib, tibia. Bar, 100 µm.
**Fig. 5.** Expression of BMP-regulated genes within 18.5 dpc lower tooth bud. Consecutive cryoections through the lower tooth bud region of a 18.5 dpc mouse embryo hybridized with a HSP47 (b), ATX (c), and **29A** (d) antisense riboprobe are shown. Expression of HSP47 is most prominent in odontoblasts and the alveolar bone (b). In contrast ATX expression is enhanced in the stratum intermedium layer, separating the ameloblast layer from the stellate reticulum (c). A lower level of expression is visible in mesenchymal tissue adjacent to tooth bud (c). Expression of **29A** is most prominent in the outer enamel layer, but also visible in the ameloblasts (d). alb, alveolar bone; amb, ameloblasts; dep, dental papillae; mes, mesenchyme; odb, odontoblasts; oen. outer enamel epithelium; str, stratum intermedium. Bar, 100 µm.
**Fig.6.** Structure of the **29A** cDNA and its expression profile in primary osteoblasts. a. Sequence of the **29A** cDNA. The first open region reading frame gives rise to a putative secreted 22 kDa protein. b. Features of the BMP-upregulated cDNAs ATX and **29A** are schemetically represented. C. **29A** is expressed *in vitro* during the osteoblast developmental sequence in cultivated primary osteoblasts isolated from 5 day old mice
**Fig. 7.** Expression of **29A** in mouse development. Parasagittal cryosections of 12.5 (e,f), 14.5 (a,b,g,h) and 16.5 (c,d,i.k) mouse embryos hybridized with a **29A** antisense riboprobe are shown. Enhanced expression of **29A** is visible in precanilage condensations of the vertebrae at 12.5 dpc (e.f). At 14.5 dpc, expression is restricted to the perichondreum of the forming vertebrae (g,h) but also visible within cartilage of the hind limb buds and ribs (b), and the faciae surrounding the spinal ganglia (arrow in b and h). Enhanced expression is in addition visible at the forming vibrissae (a.b). At 16.5 dpc expression remains enhanced in the perichondreum of the vertebrae within the tail (c,d), and the perichondreum and connective tissue sheet of the metatarsales and phalanges of the hind limb (i,k), but also at the vibrissae (c.d). bl, bladder: cts. connective tissue sheet: gt. gut: hlb. hind limb bud: li, liver: mta. mctatarsale; my, myelencephalon; per, perichondreum: pha. phalanges: sga, spinal ganglia; sk, skin; ta, tail: te. telencephalon; ve, vertebra: vi. vibrissea. Bar. ad. 1 mm: e-k. 100 µm.

**Table I**

| Days after confluence | | 0 | 4 | 7 | 9 | 11 |
|---|---|---|---|---|---|---|
| BMP-2 | Osteoblastic | 0 | 0-1 | 20 | 220 | 1100 |
| | Chondroblastic | 0 | 0 | 20 | 45 | 45 |
| | Adipocytic | 0 | 0 | 0 | covered | covered |
| | Total number of cells | 0. 19x10⁶ | 0.53x10⁶ | 0.66x10⁶ | 0.66x10⁶ | 0.68x10⁶ |
| BMP-4 | Osteoblastic | 0 | 0 | 2 | 20 | 120 |
| | Chondroblastic | 0 | 0 | 0 | 2 | 2 |
| | Adipocytic | 0 | 0 | 0 | covered | covered |
| | Total number of cells | 0.16x10⁶ | 0.26x10⁶ | 0.42x10⁶ | 0.44x10⁶ | 0.48x10⁶ |
| BMP-5 | Osieoblastic | 0 | 0 | 1 | 5 | 20 |
| | Chondroblastic | 0 | 0 | 5 | 20 | 100 |
| | Adipocytic | 0 | 0 | 0 | 100 | 500 |
| | Total number of cells | 0.13x10⁶ | 0.22x10⁶ | 0.23x10⁶ | 0.22x10⁶ | 0.22x10⁶ |
| BMP-6 | Osteoblastic | 0 | 0-2 | 110 | 1100 | 2250 |
| | Chondroblastic | 0 | 0 | 0 | 120 | 210 |
| | Adipocytic | 0 | 0 | 0 | covered | covered |
| | Total number of cells | 0.14x10⁶ | 0.4x10⁶ | 0.37x10⁶ | 0.42x10⁶ | 0.48x10⁶ |
| BMP-7 | Osteoblastic | 0 | 0 | 2 | 10 | 20 |
| | Chondroblastic | 0 | 0 | 50 | 100 | 100 |
| | Adipocytic | 0 | 0 | o | covered | covered |
| | Total number of cells | 0.21x10⁶ | 0.17x10⁶ | 0.37x10⁶ | 0.35x10⁶ | 0.36x10⁶ |
| 10T½ | Total number of cells | 0.15x10⁶ | 0.15x10⁶ | 0.17x10⁶ | 0.17x10⁵ | 0.18x10⁶ |

**Table II**

| cDNAs isolated with homology to: | Representation in the subtracted library |
|---|---|
| ApoE | |
| Autotaxin (ATX) | |
| Basigin | |
| C/EBPα | |
| C/EBPβ* | |
| C/EBPδ* | |
| Collagen (I) | |
| Collagen (II) | 8% |
| Collagen (III) | |
| Cystatin C | |
| Enolase (2-phospho-D-Glycerate-Hydrolase) | 20% |
| G0S2 | |
| Glycerolaldehydedehydrogenase (GAPDH) / Uracyl-DNA-Glykosylase (UDG) | 10% |
| HSP47 | |
| Lipoproteinlipase | |
| Migration Inhibitory Factor (MIF) | 10% |
| Osteopontin | |
| Phosphofructokinase (PFK) | |
| Phosphoglyceratekinase (PGAM) | |
| Pyruvatekinase (PK) | |
| Tropoelastin | |
| Vimentin | |
| 29A | |

## Claims

1. ssDNA
(a) having a sequence of from position 1 to 1753 according to **Fig. 6a** or
(b) having a truncated sequence compared with the ssDNA according to (a), wherein its nucleotides are within the range of from position 1 to 1753 according to **Fig. 6a** or
(c1) having a sequence which differs from that of the ssDNA according to (a) but has the same number of nucleotides or
(c2) having a sequence which differs from that of the ssDNA according to (b) but has the same number of nucleotides
wherein the ssDNA according to (c1) or (c2) is hybridizable with that according to (a) and (b), respectively.

2. A ssDNA according to claim 1
(a) having a sequence of from position 417 to 1022 or
(b) having a sequence of from position 489 to 1022.

3. ssDNA according to claim 1 (c1) or claim 1 (c2) or having a sequence which differs from that of a ssDNA according to claim 2 but has the same number of nucleotides, each being hybridizable at a temperature of at least 25 °C and a 1M sodium chloride concentration.

4. A ssDNA, **characterized** in that it is complementary to a ssDNA according to any of the preceding claims.

5. dsDNA, consisting of a ssDNA according to any of the preceding claims and its complementary strand.

6. Vector, comprising a dsDNA according to claim 5.

7. An expression product of a vector according to claim 6.

8. A cell comprising a vector according to claim 6.

9. Use of a vector according to claim 6 or an expression product according to claim 7 for a therapeutical regulation of bone growth, cartilage growth, tissue regeneration, tissue remodelling or wound healing.

10. Use of a vector according to claim 6 or an expression product according to claim 7 or a cell according to claim 8 for the detection of agonists and/or antagonists of bone, cartilage or tissue growth regulation.

11. Use of a ssDNA according to any of claims 1 to 4, a dsDNA according to claim 5 or an expression product according to claim 7 for diagnostic tests.
